# EUROPÄISCHE PATENTSCHRIFT

(11) **EP 1 415 622 B1**
(45) Veröffentlichungstag und Bekanntmachung des Hinweises auf die Patenterteilung: **06.07.2005**
(21) Anmeldenummer: 03021883.8
(22) Anmeldetag: 27.09.2003
(51) Int. Cl.: A61F 2/44

(54) **Spreizimplantat zur Anordnung zwischen Wirbeln der Wirbelsäule**
Spreading implant for positioning between two vertebral bodies of the spine
Implant écarteur pour positionner entre deux vertèbres de la colonne vertébrale

(30) Priorität: 16.10.2002 DE 10248170
(43) Veröffentlichungstag der Anmeldung: 06.05.2004
(73) Patentinhaber: Advanced Medical Technologies AG, 66620 Nonnweiler-Braunshausen (DE)
(72) Erfinder: Kast, Erich, Dr.med., 8422 Pfungen (CH); Weiland, Peter, 66620 Nonnweiler-Braunshausen (DE)
(74) Vertreter: Bernhardt, Winfrid, Dr.-Ing.

(56) Entgegenhaltungen:
- FR-A- 2 771 282
- FR-A- 2 803 741
- US-A- 6 117 174
- US-A1- 2002 068 976
- US-B1- 6 454 807

## Beschreibung

Die Erfindung betrifft ein Implantat zur Anordnung zwischen Wirbeln der Wirbelsäule, mit zwei an einem Ende miteinander verbundenen, jeweils gegen einen der Wirbel anlegbaren Schenkeln, wobei in der Ausgangsstellung für die Distraktion die den Wirbeln zugewandten Außenseiten der Schenkel sowie wenigstens abschnittsweise deren Innenseiten zu den freien Schenkelenden hin konvergieren, sowie mit einem zwischen den Schenkeln angeordneten Spreizelement zur Aufspreizung der Schenkel.

Implantate solcher Art werden nach Bandscheibenresektionen verwendet, um die betroffenen Wirbel miteinander zu verbinden. Dabei dient das Implantat vorerst als Abstandhalter, welcher den vorher von der Bandscheibe ausgefüllten Zwischenraum ausfüllt. Indem das vorzugsweise mit Durchbrüchen versehene Implantat danach von Knochengewebe durchwachsen und darin eingebettet wird, erfüllt es ferner eine Verbindungsfunktion. Das Implantat fördert die Bildung des die Wirbelkörper verbindenden Knochengewebes. Solche Implantate werden vor allem in Bereichen der Wirbelsäule eingesetzt, in denen die einander zugewandten Wirbelkörperendflächen zueinander geneigt sind. Das in den Wirbelzwischenraum eingeführte Implantat wird aufgespreizt, wobei eine Anpassung des Implantats an die zueinander geneigten Wirbelkörperendflächen erfolgt.

Implantate der eingangs erwähnten Art sind aus der FR 2 803 741 A, der US 6 454 807 B1 und der FR 2 771 282 A bekannt. Diese bekannten Implantate weisen jeweils ein mit einem Gewinde versehenes Spreizelement auf, das zur Aufspreizung der beiden Schenkel in einer Schraubbewegung vorzuschieben ist.

Der vorliegenden Erfindung liegt die Aufgabe zugrunde, ein Implantat der eingangs erwähnten Art zu schaffen, das sich leichter als bekannte solche Implantate handhaben lässt.

Das diese Aufgabe lösende Implantat nach der Erfindung ist dadurch gekennzeichnet, dass das Spreizelement als ausschließlich translatorisch bewegbarer Schieber ausgebildet ist, welcher die Schenkel im aufgespreizten Zustand über ihre gesamte horizontale Breite abstützt.

Vorteilhaft braucht zur Aufspreizung der Schenkel nur eine Translationsbewegung des Schiebers zu erfolgen.

Zweckmäßig sind die miteinander verbundenen Schenkel aus einem einzigen, zur Distraktion verformbaren Kunststoffmaterialstück hergestellt, z.B. Polyetheretherketon (PEEK). Auch Titan kommt hierfür in Betracht. Vorteilhaft besteht auch der Schieber, der in einer Endstellung, in welcher die Schenkel gespreizt sind, als Abstandhalter zwischen den Schenkeln im Implantat verbleibt, aus diesem hoch belastbaren Kunststoffmaterial.

Zweckmäßig ist der Schieber in der genannten Endstellung zur Außenfläche des Implantats bündig, d.h. er fügt sich ohne Absätze in dessen Außenkonturen ein.

In der bevorzugten Ausführungsform der Erfindung ist das Implantat zur Anordnung in einen seitlichen Halbraum eines nach einer Bandscheibenresektion auszufüllenden Wirbelzwischenraums vorgesehen. Zur Verbindung der Wirbel bedarf es zwei solcher, zueinander spiegelsymmetrischer Implantate. Vorteilhaft lassen sich solche schmalen Implantate vom Rücken her durch den Nervenkanal der Wirbelsäule unter Vorbeiführung am Nervenstrang mit verhältnismäßig geringem Aufwand implantieren.

Zweckmäßig ist an der den freien Schenkelenden gegenüberliegenden Seite des Implantats eine Öffnung für die Durchführung eines Werkzeugs insbesondere zur Betätigung des Schiebers gebildet. In weiterer vorteilhafter Ausgestaltung der Erfindung rastet der Schieber in seiner Endstellung ein, wobei vorteilhaft eine lösbare Rastung gebildet ist, die es erlaubt, die Aufspreizung z.B. zwecks genauerer Positionierung des Implantats zwischen den Wirbeln vorübergehend rückgängig zu machen.

Zweckmäßig weist das Implantat in grober Näherung in seinen Umrissen eine Quaderform auf. Insbesondere die den Wirbeln zugewandten Seiten können jedoch an die Form der einander zugewandten Wirbeloberflächen angepasst sein und das Implantat in Richtung von vorn nach hinten ein Höhenmaximum aufweisen, während die Höhe von der Wirbelmitte zum seitlichen Wirbelrand hin abfällt.

Die Erfindung soll nun anhand eines Ausführungsbeispiels und der beiliegenden, sich auf dieses Ausführungsbeispiel beziehenden Zeichnungen näher erläutert werden. Es zeigen:
- Fig. 1: eine Teildarstellung eines spreizbaren Implantats nach der Erfindung in einer perspektivischen Ansicht,
- Fig. 2: das um ein Spreizelement ergänzte Implantat von Fig. 1 in einer Draufsicht,
- Fig. 3: das gemäß Fig. 2 ergänzte Implantat in einer Seitenansicht im ungespreizten Zustand, und
- Fig. 4: die Seitenansicht gemäß Fig. 3 im gespreizten Zustand des Implantats.

Das in den Figuren gezeigte, in grober Näherung quaderförmige Implantat weist eine Oberseite 1, eine Unterseite 2, Längsseiten 3 und 4 sowie Stirnseiten 5 und 6 auf. Die Ecken und Kanten des Implantats sind abgerundet.

In dem gezeigten Ausführungsbeispiel besteht das Implantat aus Polyetheretherketon (PEEK).

Eine Abweichung von der Quaderform besteht darin, dass die Implantathöhe von der Stirnseite 5 zur Stirnseite 6 hin bis zu einem Maximum 7 ansteigt und dann wieder abfällt, wobei das Maximum im letzten Drittel der Strecke zwischen den Stirnseiten 5 und 6 liegt.

In weiterer Abweichung von der Quaderform fällt die Höhe des Implantats von der Längsseite 3 zur Längsseite 4 hin leicht ab. Symmetrie besteht nur in bezug auf eine das Implantat in der Höhenmitte schneidende horizontale Ebene.

Das Implantat dient zur Ausfüllung eines seitlichen Halbraums zwischen zwei Wirbelkörpern. Im gegenüberliegenden Halbraum wäre ein zu diesem Implantat spiegelsymmetrisches Implantat zu implantieren. Die Implantation erfolgt in Richtung eines Pfeils 8 vom Rücken her durch den Nervenkanal der Wirbelsäule hindurch vorbei am Nervenstrang.

Das Implantat weist einen horizontalen Durchbruch 9 und einen vertikalen Durchbruch 10 auf, wobei sich die als Langlöcher ausgebildeten Durchbrüche 9 und 10 kreuzen. An der Stirnseite 5 ist eine weitere, nach innen zu den Durchbrüchen 9 und 10 öffnende Schlitzausnehmung 11 vorgesehen.

Durch den horizontalen Durchbruch 9 und die Schlitzausnehmung 11 sind vertikal bewegliche Schenkel 12 und 13 gebildet, die über einen Steg 14 an der Stirnseite 6 miteinander verbunden sind.

Der Steg 14 weist eine Durchgangsbohrung 15 mit einer Schlitzansenkung 16 auf. Die Durchgangsbohrung 15 öffnet nach innen sowohl zu dem horizontalen Durchbruch 9 als auch dem vertikalen Durchbruch 10.

Neben der Durchgangsbohrung 15 ist senkrecht eine weitere Durchgangsbohrung 17 in dem Steg 14 für die Aufnahme eines Metallstifts gebildet. An der Ober- und Unterseite des Implantats ist jeweils eine Zahnung 18 vorgesehen, deren Zahnkämme sich quer zur Längsrichtung des Implantats von der Längsseite 4 bis zu dem vertikalen Durchbruch 10 erstrecken.

Mit dem Bezugszeichen 19 ist ein zwischen den Schenkeln 12 und 13 angeordneter, ein Spreizelement bildender Schieber bezeichnet, welcher bündig mit den Längsseiten 3 und 4 des Implantats abschließt. Auch der Schieber ist wie das die Schenkel 12 und 13 sowie den Steg 14 bildende Materialstück einstückig aus PEEK hergestellt.

Von der Ober- und Unterseite des Schiebers 19 steht jeweils ein Führungszapfen 20 vor, welcher in den oberen bzw. unteren Abschnitt des vertikalen Durchbruchs 10 eingreift.

Bei Verschiebung in Richtung zu den freien Enden der Schenkel 12 und 13 kommt der Schieber 19 gegen konvergierende, einander gegenüberliegende Innenseiten 21 und 22 der Schenkel 12 und 13 zur Anlage. Von den Innenseiten 21 und 22 steht jeweils eine Rastnase 23 vor.

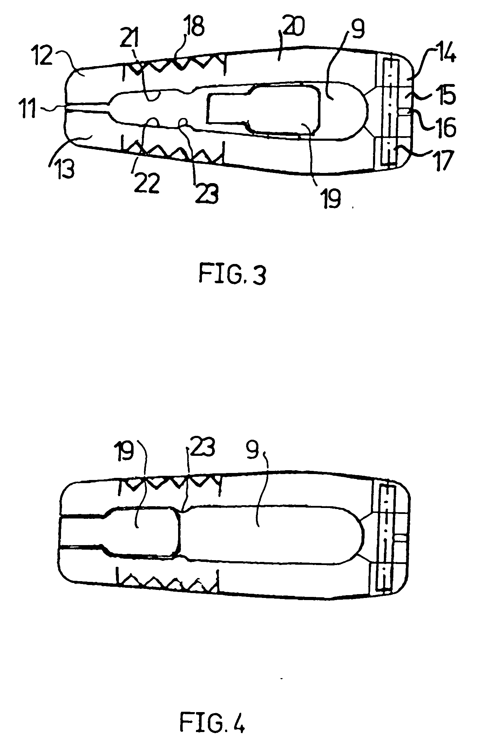

Bei der Implantation befindet sich der Schieber 19 zunächst etwa in der in Fig. 3 gezeigten Position. Das die Schenkel 12 und 13 sowie den Steg 14 bildende Materialstück ist in diesem Zustand unverformt. Die Schenkel 12 und 13 bilden einen stumpfen Keil, der es erleichtert, das Implantat in den Zwischenraum zwischen zwei Wirbeln einzuführen.

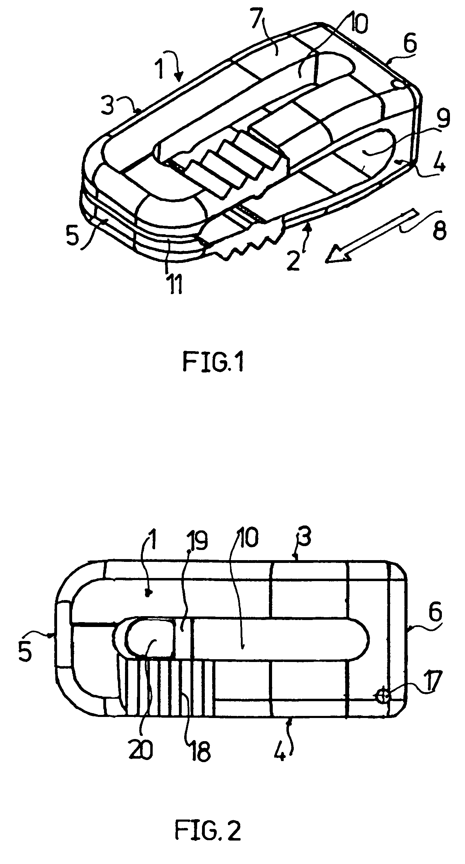

In der Implantationsposition zwischen den Wirbeln wird der Schieber 19 mit Hilfe eines durch die Durchgangsbohrung 15 geführten, ggf. in die Schlitzeinsenkung eingreifenden Werkzeugs in Richtung zu den freien Schenkelenden verschoben, wobei der gleitbeweglich gegen die konvergierenden Innenseiten 21 und 22 anliegende Schieber die Schenkel 12 und 13 aufspreizt. Wie insbesondere Fig. 2 zu entnehmen ist, liegt der Führungszapfen 20 mit Seitenflächen gegen die Innenwand des vertikalen Durchbruchs 10 an, so dass der Schieber 19 zwischen den Schenkeln 12 und 13 verdrehsicher geführt ist.

In der in Fig. 4 gezeigten Endstellung ist der Schieber 19 hinter den Rastnasen 23 eingerastet. In dieser Stellung fügt er sich in das Implantat bündig mit dessen Außenfläche ein und bildet auf der Stirnseite 5 einen Abstandhalter, der das Implantat in der in Fig. 4 gezeigten Form festhält, in welcher das die Schenkel 12 und 13 sowie den Steg 14 bildende einstückige Kunststoffteil elastisch verformt ist.

Der Schieber 19 könnte an seiner der Durchgangsbohrung 15 zugewandten Seite für den Angriff eines Werkzeugs geeignete Vertiefungen oder Vorsprünge insbesondere derart aufweisen, dass er sich aus der in Fig. 4 gezeigten Position zurückziehen lässt, z.B. um das Implantat lösen und zwischen den Wirbelkörpern in einer neuen Position platzieren zu können.

Durch die Bohrung 15 läßt sich auch ein Werkzeug führen, mit dessen Hilfe in den dem Durchbruch 10 gegenüberliegenden Wirbelbereichen Knochenweichgewebe frei gelegt werden kann. Das Knochenweichgewebe bildet dann neues, den Durchbruch 10 durchsetzendes Knochengewebe.

In dem in Fig. 4 gezeigten Zustand dringt die Zahnung 18 in gegen das Implantat anliegendes Knochenhartgewebe ein, wodurch eine sichere Arretierung des Implantats zwischen den Wirbelkörpern erreicht wird.

Zweckmäßig liegt die Zahnung 18 so weit wie möglich vom Nervenkanal und der Hauptbelastungsachse der Wirbelsäule entfernt, so dass durch das Eindringen in das Knochengewebe weder Nervenbahnen verletzbar sind noch die Tragfähigkeit der Wirbelsäule beeinträchtigt wird.

In dem gezeigten Ausführungsbeispiel ist die Implantathöhe an der Stirnseite 5 im aufgespreizten Zustand so groß wie an der gegenüberliegenden Stirnseite 6. Die den Wirbelkörpern zugewandten Außenseiten der Schenkel könnten in ihrem Dickenprofil in Längsrichtung des Implantats jedoch auch so ausgebildet sein, dass die Implantathöhe im gespreizten Zustand an der Stirnseite 5 größer oder kleiner als an der Stirnseite 6 ist, um eine Anpassung an zueinander geneigte Wirbel insbesondere im lumbalen Bereich der Wirbelsäule zu erreichen.

Der Schieber 19 könnte eine zu den Längsseiten 3 und 4 öffnende Durchgangsbohrung für die Aufnahme eines weiteren, zu dem Metallstift in der Bohrung 17 senkrecht angeordneten Metallstifts aufweisen, so dass anhand zweier solcher, sich im Röntgenbild gut abhebender Stifte die räumliche Position des Implantats genau beurteilt werden kann.

Die zur Führung des Schiebers 19 dienenden Durchbrüche 9 und 10 können nach der Implantation vom Knochengewebe durchwachsen werden, wobei das Implantat weitgehend in die Wirbel miteinander verbindendes Knochengewebe eingebettet wird.

## Patentansprüche

1. Implantat zur Anordnung zwischen Wirbeln der Wirbelsäule, mit zwei an einem Ende miteinander verbundenen, jeweils gegen einen der Wirbel anlegbaren Schenkeln (12,13), wobei in der Ausgangsstellung für die Distraktion die den Wirbeln zugewandten Außenseiten der Schenkel sowie wenigstens abschnittsweise deren Innenseiten zu den freien Schenkelenden hin konvergieren, sowie mit einem zwischen den Schenkeln angeordneten Spreizelement zur Aufspreizung der Schenkel (12,13),
**dadurch gekennzeichnet,**
**dass** das Spreizelement als ausschließlich translatorisch bewegbarer Schieber (19) ausgebildet ist, welcher die Schenkel (12,13) im aufgespreizten Zustand über ihre gesamte horizontale Breite abstützt.

2. Implantat nach Anspruch 1,
**dadurch gekennzeichnet,**
**dass** die miteinander verbundenen Schenkel (12,13) aus einem einzigen, zur Distraktion verformbaren Materialstück, vorzugsweise aus einem Kunststoff, hergestellt sind.

3. Implantat nach Anspruch 2,
**dadurch gekennzeichnet,**
**dass** der Kunststoff Polyetheretherketon (PEEK) ist.

4. Implantat nach einem der Ansprüche 1 bis 3,
**dadurch gekennzeichnet,**
**dass** an der den freien Schenkelenden gegenüberliegenden Seite des Implantats eine Öffnung (16) für die Durchführung eines ggf. den Schieber (19) betätigenden Werkzeugs gebildet ist.

5. Implantat nach einem der Ansprüche 1 bis 4,
**dadurch gekennzeichnet,**
**dass** der Schieber (19) in einer Endstellung, in der die Schenkel (12,13) gespreizt sind, vorzugsweise lösbar, einrastet.

6. Implantat nach einem der Ansprüche 1 bis 5,
**dadurch gekennzeichnet,**
**dass** der Schieber in einer Endstellung, in der die Schenkel (12,13) gespreizt sind, sich in das Implantat bündig zu dessen Außenfläche einfügt.

7. Implantat nach einem der Ansprüche 1 bis 6,
**dadurch gekennzeichnet,**
**dass** das Implantat zur Anordnung in einem seitlichen Halbraum zwischen zwei Wirbeln in Verbindung mit einem dazu spiegelsymmetrischen, in dem anderen Halbraum angeordneten solchen Implantat vorgesehen ist.

8. Implantat nach einem der Ansprüche 1 bis 7,
**dadurch gekennzeichnet,**
**dass** der Schieber in einem als Langloch ausgebildeten vertikalen Durchbruch des Implantats geführt ist.

## Claims

1. Implant for placement between vertebrae of the spinal column, with two branches (12, 13) which are connected to one another at one end and can each bear against one of the vertebrae, where, in the starting position for distraction, the outer faces of the branches directed towards the vertebrae and, at least in some areas, their inner faces converge towards the free ends of the branches, and with a spreader element arranged between the branches for spreading the branches (12, 13) apart, **characterized in that** the spreader element is designed as a slide (19) which can move exclusively in translation and which supports the branches (12, 13), in the spread-apart state, over their entire horizontal width.

2. Implant according to Claim 1, **characterized in that** the interconnected branches (12, 13) are made as a single material piece which is deformable for distraction, preferably made of plastic.

3. Implant according to Claim 2, **characterized in that** the plastic is polyetheretherketone (PEEK).

4. Implant according to one of Claims 1 to 3, **characterized in that** an opening (16) is formed on that side of the implant remote from the free ends of the branches and permits passage of a tool which can operate the slide (19).

5. Implant according to one of Claims 1 to 4, **characterized in that** the slide (19) locks, preferably releasably, in an end position in which the branches (12, 13) are spread.

6. Implant according to one of Claims 1 to 5, **characterized in that**, in an end position in which the branches (12, 13) are spread, the slide passes into the implant, flush with the outer face thereof.

7. Implant according to one of Claims 1 to 6, **characterized in that** the implant is provided for placement in a lateral half-space between two vertebrae, in conjunction with a similar mirror-symmetrical implant placed in the other half-space.

8. Implant according to one of Claims 1 to 7, **characterized in that** the slide is guided in a vertical passage of the implant configured as an oblong hole.

## Revendications

1. Implant destiné à être disposé entre des vertèbres de la colonne vertébrale, présentant deux branches (12, 13) liées l'une à l'autre en une extrémité, pouvant être placées à chaque fois contre une des vertèbres, où, dans la position de départ pour la distraction, les faces externes des branches orientées vers les vertèbres ainsi qu'au moins en partie leurs faces internes convergent vers les extrémités libres des branches, ainsi qu'un élément écarteur disposé entre les branches destiné à écarter les branches (12, 13), **caractérisé en ce que** l'élément écarteur est réalisé sous forme d'un curseur (19) pouvant être déplacé exclusivement par translation, qui supporte les branches (12, 13) à l'état écarté sur toute leur largeur horizontale.

2. Implant selon la revendication 1, **caractérisé en ce que** les branches (12, 13) liées l'une à l'autre sont réalisées en une seule pièce en un matériau pouvant être déformé pour la distraction, de préférence en un matériau synthétique.

3. Implant selon la revendication 2, **caractérisé en ce que** le matériau synthétique est la polyétheréthercétone (PEEC).

4. Implant selon l'une quelconque des revendications 1 à 3, **caractérisé en ce qu'**une ouverture (16) est réalisée au niveau du côté opposé aux extrémités libres des branches de l'implant, destinée à y faire passer un outil actionnant le cas échéant le curseur (19).

5. Implant selon l'une quelconque des revendications 1 à 4, **caractérisé en ce que** le curseur (19) se verrouille dans une position finale dans laquelle les branches (12, 13) sont écartées, de préférence de manière déverrouillable.

6. Implant selon l'une quelconque des revendications 1 à 5, **caractérisé en ce que** le curseur, dans une position finale dans laquelle les branches (12, 13) sont écartées, s'insère dans l'implant en étant affleurant à ses surfaces extérieures.

7. Implant selon l'une quelconque des revendications 1 à 6, **caractérisé en ce que** l'implant est prévu pour être disposé dans un demi-espace latéral entre deux vertèbres, en association avec un autre implant identique disposé de manière symétrique dans un miroir dans l'autre demi-espace.

8. Implant selon l'une quelconque des revendications 1 à 7, **caractérisé en ce que** le curseur est guidé dans un trou vertical de l'implant réalisé sous forme d'une ouverture longitudinale.
